# EUROPEAN PATENT APPLICATION

(11) **EP 4 431 183 A1**
(43) Date of publication of application: **18.09.2024**
(21) Application number: 21963520.8
(22) Date of filing: 09.11.2021
(51) Int. Cl.: B01L 3/00, C12M 1/00

(54) **LIQUID PATH SYSTEM, GENE SEQUENCER, AND REAGENT RECOVERY METHOD**

(71) Applicant: MGI Tech Co., Ltd., Shenzhen, Guangdong 518083 (CN)
(72) Inventor: LU, Hao, Shenzhen, Guangdong 518083 (CN); NIU, Zihua, Shenzhen, Guangdong 518083 (CN); LI, Songlin, Shenzhen, Guangdong 518083 (CN); XING, Chutian, Shenzhen, Guangdong 518083 (CN)
(74) Representative: Inspicos P/S
(86) International application number: PCT/CN2021/129640
(87) International publication number: WO 2023/082063

(57) **Abstract**

A flow path system, a gene sequencer and a reagent recovery method. The flow path system comprising at least two reagent storage containers, a flow cell, a shunting module and a fluid power unit. The flow cell is connected to the at least two reagent storage containers. The shunting module comprises a shunt structure and at least two shunt channels. The fluid power unit is connected to the shunting module, the fluid power unit is selectively connected to one of the at least two shunt channels, and the fluid power unit is configured to drive a forward flow of the reagent from the reagent storage container toward the shunting module, and the fluid power unit is further configured to drive a reverse flow of the reagent from the shunting module toward the reagent storage container.

## Description

### Technical Field

The present application relates to a flow path system, a gene sequencer and a reagent recovery method.

### Background

Various testing instruments used for clinical diagnosis and life science research (such as gene sequencers, flow cytometers, and high-pressure liquid chromatographs) usually have a fluid system to transport different solutions among multiple areas. For example, samples containing detection objects (cells, DNA fragments, etc.), biochemical reagents that react with the samples, various buffers, cleaning solutions, etc. are generally transported to one or more reaction areas or detection areas from sample tubes, reagent cells and other containers to one or more reaction areas or detection areas, and then transferred to a waste liquid area after the reaction or detection is completed. The concentration and uniformity of reagents entering the reaction area determine the efficiency of biochemical reactions, and the former is closely related to the overall design of the fluid system. In addition, samples and biochemical reagents are often precious and costly, which means that the amount of reagents used during transportation must be small enough.

At present, the fluid systems of high-end testing instruments mostly adopt the form of pressure drive, using high-precision, low-internal volume pump valve components and pipes to fluidly connect the above-mentioned reagent storage area, reaction area or detection area, waste liquid area and other areas. Since biochemical reactions are often carried out step by step, different reagents need to pass through the reaction area in sequence, which in turn involves the problem of substitution between reagents. For the flow in a closed pipeline or channel (also known as Poiseuille flow), the viscosity of the fluid causes a low flow velocity close to the wall and a high flow velocity far away from the wall, and the overall velocity is distributed parabolically. Therefore, when one reagent in a section of pipeline is replaced with or flushed by the following reagent, the reagent in the area close to the wall is often more difficult to replace, resulting in the need to use an amount of reagent several times the volume of the pipeline to flush the pipeline.

Specifically, if you want a pipeline or flow cell with a volume V and full of a certain reagent a to be completely filled with another reagent b, the required amount of reagent b should be at least rV, where r>1 is defined as the substitution ratio, r is a number related to many variables, such as pipeline geometry, viscosity ratio and density ratio of reagents a and b, flow rate, etc. Experiments and numerical simulations jointly show that for a straight pipeline with a circular cross-section, r is usually between 4 and 5, and for a channel having a height much less than its length and width (for example, the length and width are 100 times the height), r is between 1.5 and 2. These two basic shapes are very common in fluid systems of medical testing instruments. For example, in a gene sequencer, DNA fragments to be tested are usually fixed on a flow cell. In order to reduce the internal volume of the flow cell as much as possible to reduce the amount of reagents required, and at the same time ensure that the DNA fragments to be tested are spread on a detection plane as much as possible, the typical height dimension of the flow cell channel is generally only 50 to 100 microns, which is much less than the length and width (length and width constitute the detection plane) in millimeters or centimeters. In addition, the flow cell is fluidly connected to reagent cells, waste liquid cells, pump valve components, and the like at the upper and lower reaches of the flow cell, usually using standard round pipes that are easy to process. Now assume that there is a flow cell with a volume Vi, and its upper reach is smoothly connected to a section of a round pipeline with an internal volume V₂. Both the flow cell and the round pipeline are full filled with reagent a. For the convenience of calculation, the r value of the flow cell is fixed at 2 and the r value of the round pipeline is fixed at 5. At this time, if reagent b is injected into an inlet end of the round pipeline, 5V₂ of reagent b is needed to completely replace reagent a in the round pipe, and 2V₁ of reagent b is needed to further completely replace reagent a in the flow cell. Considering that during the replacement process of the round pipeline at the upper reach, some reagent b has entered the flow cell, the final volume of reagent b used is generally between 2V₁ and 2V₁+5V₂. It can be seen that the characteristics of Poiseuille flow make the amount of reagents used not only depend on the internal volume V₁ of the detection area, but also highly related to the internal volume V₂ of the pipeline at the upper reach of the detection area. Especially for some fluid system where the internal volume of the detection area is smaller than the volume of the pipeline at the upper reach (i.e., V₁ < V₂), a large amount of reagents are required to ensure the reagent concentration and reaction efficiency in the detection area. In order to reduce the amount of reagents used, an obvious solution is to reduce the volume or relative volume of the pipeline at the upper reach (that is, reduce V₂ or V₂/V₁), which can be achieved by reducing the cross-sectional area or length of the pipe. However, the reduction in the cross-sectional area of the pipeline will inevitably lead to an increase in the overall pressure drop, thereby increasing the load on the fluid system. The pipeline length has a lower limit due to the constraints of the physical space inside the instrument. In short, V₂ or V₂/V₁ cannot be reduced to zero.

In addition, another technical solution that can effectively reduce the amount of reagents used is reagent recovery. How to avoid cross-contamination caused by other reagent being recovered into the flow cell during reagent recovery is a problem that needs to be solved.

### Summary

The present application provides a flow path system, a gene sequencer and a reagent recovery method to avoid cross-contamination caused by the reagent of the previous reaction being recovered into the flow cell.

In a first aspect, the present application provides a flow path system, including at least two reagent storage containers, a flow cell, a shunting module and a fluid power unit, wherein the at least two reagent storage containers are configured to store at least two different reagents respectively. The flow cell is configured to accommodate samples, and the flow cell is fluidly connected to the at least two reagent storage containers. The shunting module includes a shunt structure and at least two shunt channels, the shunt structure has a converging port communicating with the flow cell and at least two shunt ports corresponding to the at least two shunt channels. The fluid power unit is fluidly connected to the shunting module, the fluid power unit is selectively being in fluid communication with one of the at least two shunt channels, and the fluid power unit is configured to drive a forward flow of the reagent from the reagent storage container toward the shunting module, and the fluid power unit is further configured to drive a reverse flow of the reagent from the shunting module toward the reagent storage container.

In some embodiments, the at least two shunt channels and the at least two reagent storage containers are provided in one-to-one correspondence.

In some embodiments, the at least two shunt channels include a first shunt channel and a second shunt channel, the shunt structure includes a three-way pipe, and the three-way pipe includes a converging port fluidly connected to the flow cell, a first shunt port fluidly communicated with the first shunt channel, and a second shunt port fluidly communicated with to the second shunt channel.

In some embodiments, the shunt structure further includes an on-off control valve, and the on-off control valve is provided on the first shunt channel and/or the second shunt channel.

In some embodiments, the at least two shunt channels include a first shunt channel and a second shunt channel, the shunt structure includes a first reversing valve, the first reversing valve has a first port, a second port and a third port, the first port of the first reversing valve forms a converging port, the second port of first reversing valve forms a first shunt port fluidly communicated with the first shunt channel, the third port of the first reversing valve forms a second shunt port fluidly communicated with the second shunt channel, and the first reversing valve acts to control the communication of the first port to the second port or the third port.

In some embodiments, the fluid power unit includes an injection pump, the injection pump includes a first power port and a second power port, the first power port is fluidly connected to the first shunt channel, and the second power port is fluidly connected to the second shunt channel.

In some embodiments, the flow path system further includes a waste liquid cell, the injection pump further includes a third power port, and the third power port is fluidly communicated with the waste liquid cell.

In some embodiments, the fluid power unit includes an injection pump and a second reversing valve, the injection pump includes a first power port, the second reversing valve has a first port, a second port and a third port, the first port and the second port are connected to the first shunt channel and the second shunt channel respectively, the third port is connected to the first power port of the injection pump, and the second reversing valve acts to control the communication of the third port to the first port or the second port.

In some embodiments, the flow path system further includes a waste liquid cell, the injection pump further includes a second power port, and the second power port of the injection pump communicates with the waste liquid cell.

In some embodiments, the fluid power unit includes a first peristaltic pump and a second peristaltic pump, the flow path system further includes a waste liquid cell, the first shunt channel and the second shunt channel both communicates with the waste liquid cell, the first peristaltic pump is provided on the first shunt channel, and the second peristaltic pump is provided on the second shunt channel.

In some embodiments, the flow path system further includes a reagent selection component, the reagent selection component includes a common hole and at least two branch holes, the at least two branch holes are correspondingly fluidly connected to the at least two reagent storage containers, the common hole is fluidly connected to the flow cell, and the common hole is selectively communicated with one of the at least two branch holes.

In some embodiments, the fluid power unit includes an injection pump, the flow path system further includes a waste liquid cell and a reagent selection component, the first shunt channel and the second shunt channel both communicates with the waste liquid cell, the injection pump includes a power port, the reagent selection component includes a common hole and a plurality of branch holes, the common hole selectively communicates with one of the plurality of branch holes, the plurality of branch holes include at least two reagent branch holes correspondingly communicating with the at least two reagent storage containers and a flow cell branch hole communicating with the flow cell, and the power port of the injection pump is connected to the common hole.

In some embodiments, the flow path system further includes a buffer storage container for storing a buffer, the buffer storage container is fluidly connected to the flow cell, and the fluid power unit is configured to drive a forward flow of the buffer from the buffer storage container toward the shunting module.

In some embodiments, the fluid power unit is configured to drive a reverse flow of the reagent from the shunting module toward the reagent storage container and back into a pipeline connected to an outlet end of the reagent storage container.

In a second aspect, the present application provides a gene sequencer, including a sequencing slide and the flow path system described above, a flow cell being arranged on the sequencing slide.

In a third aspect, the present application provides a reagent recovery method based on the flow path system described above, the at least two different reagents including a first reagent and a second reagent, the at least two shunt channels including a first shunt channel and a second shunt channel, the reagent recovery method including the following steps:
controlling the fluid power unit to communicate with the first shunt channel and drive the first reagent to enter the first shunt channel of the at least two shunt channels via the flow cell and the shunt structure, wherein the first reagent has first reaction with a sample in the flow cell; and
controlling the action of the fluid power unit to drive the second reagent to flow through the flow cell and the shunt structure, wherein the second reagent has second reaction with the sample in the flow cell, and controlling the fluid power unit to drive the second reagent to flow back toward the reagent storage container after the second reaction.

In some embodiments, the reagent recovery method further includes, after the first reaction, controlling the action of the fluid power unit to drive the buffer to flow through the flow cell and the shunt structure and enter the first shunt channel to implement cleaning.

In some embodiments, the operation of controlling the action of the fluid power unit to drive the second reagent to flow through the flow cell and the shunt structure, wherein the second reagent has second reaction with the sample in the flow cell, and controlling the fluid power unit to drive the second reagent to flow back toward the reagent storage container after the second reaction includes: controlling the fluid power unit to connect with the second shunt channel and drive the second reagent to enter the second shunt channel of the at least two shunt channels via the flow cell and the shunt structure, and after the second reaction, controlling the fluid power unit to connect with the second shunt channel and drive the second reagent to flow back toward the reagent storage container.

In some embodiments, the operation of controlling the action of the fluid power unit to drive the second reagent to flow through the flow cell and the shunt structure, wherein the second reagent has second reaction with the sample in the flow cell, and controlling the fluid power unit to drive the second reagent to flow back toward the reagent storage container after the second reaction includes: controlling the fluid power unit to connect with the first shunt channel and drive the second reagent to enter the first shunt channel via the flow cell and the shunt structure, and after the second reaction, controlling the fluid power unit to connect with the second shunt channel and drive the second reagent to flow back toward the reagent storage container.

In some embodiments, the reagent recovery method further includes, after the first reaction, controlling the fluid power unit to connect with the first shunt channel and drive the first reagent to flow back toward the reagent storage container so that the recovered first reagent flows back into a pipeline connected to an outlet end of the reagent storage container that stores the first reagent.

In some embodiments, the operation of controlling the fluid power unit to drive the second reagent to flow back toward the reagent storage container includes: controlling the fluid power unit to drive the second reagent to flow back toward the reagent storage container so that the recovered second reagent flows back into a pipeline connected to an outlet end of the reagent storage container.

Based on various aspects of the present application, the flow path system is provided with a shunting module, and the shunting module includes a shunt structure and at least two shunt channels, so that the reagent that need to be recovered can enter a different shunt channel than the reagent from the previous reaction, thereby avoiding cross-contamination caused by the reagent from the previous reaction being recovered into the flow cell.

Other features and advantages of the present application will become clear from the following detailed description of exemplary embodiments of the present application with reference to the accompanying drawings.

### Brief Description of the Drawings

The accompanying drawings described herein are intended to provide a further understanding of the application and constitute a part of this application. The illustrative embodiments of the application and their descriptions are used to explain the application and do not constitute undue limitations on the application. In the attached drawings.
Fig. 1 is a schematic structural diagram of a flow path system in the prior art when a first reagent flows forward.
Fig. 2 is a schematic structural diagram of the flow path system in the prior art when the first reagent flows in a reverse direction for recovery.
Fig. 3 is a schematic structural diagram of a flow path system according to some embodiments of the present application.
Fig. 4 is a flow chart of a reagent recovery method based on an embodiment of the flow path system shown in Fig. 3.
Fig. 5 is a step diagram of a reagent recovery method according to some embodiments of the present application.
Fig. 6 is a step diagram of a reagent recovery method according to other embodiments of the present application.
Fig. 7 is a flow chart of a reagent recovery method according to some embodiments of the present application.
Fig. 8 is a schematic structural diagram of a flow path system according to first embodiment of the present application.
Fig. 9 is a diagram showing the change of the reagent concentration in a reagent storage container with the number of cycles when the flow path system shown in Fig. 8 is used for reagent recovery.
Fig. 10 is a schematic structural diagram of a flow path system according to second embodiment of the present application.
Fig. 11 is a schematic structural diagram of a flow path system according to third embodiment of the present application.
Fig. 12 is a schematic structural diagram of a flow path system according to fourth embodiment of the present application.
Fig. 13 is a schematic structural diagram of a flow path system according to fifth embodiment of the present application.
Fig. 14 is a schematic structural diagram of a flow path system according to sixth embodiment of the present application.

### Detailed Description of the Embodiments

In the following, the technical solution in the embodiment of the application will be clearly and completely described with reference to the attached drawings. Obviously, the described embodiment is only a part of the embodiment of the application, but not the whole embodiment. The following description of at least one exemplary embodiment is merely illustrative in nature and is in no way intended to limit the application, its application or uses. Based on the embodiments in this application, all other embodiments obtained by ordinary technicians in this field without creative work belong to the protection scope of this application.

Unless otherwise specified, the relative arrangement of components and steps, numerical expressions and numerical values set forth in these embodiments do not limit the scope of the present application. At the same time, it should be understood that for the convenience of description, the dimensions of various parts shown in the drawings are not drawn according to the actual scale relationship. Techniques, methods and equipment known to those skilled in the art may not be discussed in detail, but in appropriate cases, techniques, methods and equipment should be regarded as part of the authorized description. In all examples shown and discussed herein, any specific values should be interpreted as illustrative only and not as a limitation. Therefore, other examples of exemplary embodiments may have different values. It should be noted that similar numbers and letters indicate similar items in the following drawings, so once an item is defined in one drawing, it does not need to be further discussed in subsequent drawings.

For the convenience of description, spatially relative terms such as "on", "above", "on the upper surface of" and "upper" can be used here to describe the spatial positional relationship between a device or feature as shown in the FIG. and other devices or features. It should be understood that spatially relative terms are intended to encompass different orientations in use or operation in addition to the orientation of the device depicted in the drawings. For example, if the devices in the drawings are inverted, devices described as "above" or "on" other devices or structures will be positioned as "below" or "under" other devices or structures. Thus, the exemplary term "above" can include both directions of "above" and "below". The device can also be positioned in other different ways, and the spatial relative description used here is explained accordingly.

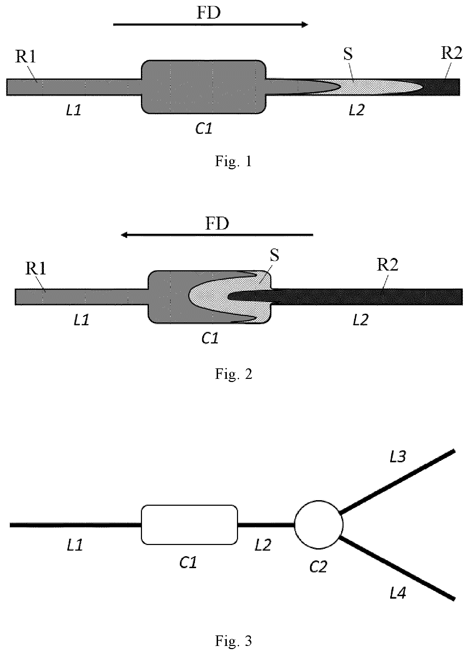

As shown in Figs. 1 and 2, the flow path system in the related art includes a flow cell C1, a first pipeline L1 and a second pipeline L2. The first pipeline L1 is connected to a first end of the flow cell C1, and the second pipeline L2 is connected to a second end of the flow cell C1. The arrow FD shows the flow direction. In the flow path system shown in Figs. 1 and 2, in each reaction cycle, the sample first has first reaction with the first reagent and then has second reaction with the second reagent, and then the above reaction cycle is repeated multiple times. Then, in a case where two reaction cycles occur, the second reagent in the first reaction cycle reacts first, and the first reagent in the second reaction cycle reacts later. Fig. 1 shows a schematic diagram of the first reagent R1 flowing forward from the first pipeline L1 to the second pipeline L2 in a certain reaction cycle. As shown in Fig. 1, when the first reagent R1 flows forward from the first pipeline L1 to the second pipeline L2, there are still, in the second pipeline L2, residues of buffer S and the second reagent R2 from the previous reaction cycle. Fig. 2 shows a schematic diagram of the first reagent R1 flowing back into the first pipeline L1 from the second pipeline L2. That is, Fig. 2 shows a schematic diagram of recovering the first reagent R1.

Based on the characteristics of Poiseuille flow, i.e., the parabolic distribution of flow velocity in a closed pipeline or channel, the interface between reagents is not a straight line, but a more complex curve. As can be seen from Fig. 1, when the first reagent R1 flows forward from left to right into the flow cell C1, the second pipeline L2 contains the buffer S replaced by the first reagent R1 and the second reagent R2 replaced earlier. In this case, the interface between the first reagent R1 and the buffer S and the interface between the buffer S and the second reagent R2 are both parabolas, and the buffer S acts as a barrier between the first reagent R1 and the second reagent R2. In this way, when part of the first reagent R1 is recovered in a reverse direction from right to left (see Fig. 2), since the flow rate in the middle of the flow cell C1 is the highest, a small amount of the second reagent R2 may flow back into the flow cell C1 along the middle of the flow cell. In some reaction systems that need to be carried out in a certain order, this is likely to cause a negative impact. For example, gene sequencing usually involves a periodic "synthesis-test-excision" process for each base on a single-stranded DNA fragment. In the synthesis stage, free bases with fluorophores enter the flow cell using a synthesis reagent as a medium, and under the action of polymerase, they are complementary to the single-stranded DNA to be tested fixed on the surface of the flow cell. In the subsequent test phase, the optical system develops the fluorophores to identify the bases of the current cycle. Finally, an excision reagent enters the flow cell to excise the fluorophore, thus ending the current cycle. In Figs. 1 and 2, assume that the first reagent R1 is the synthesis reagent and the second reagent R2 is the excision reagent in the previous cycle. When the first reagent R1 enters the flow cell C1 (Fig. 1), a new cycle of synthesis reaction begins. At this time, in the order of "synthesis-test-excision", optical development should be performed next. However, due to reagent recovery, part of the excision reagent flows back into the flow cell (Fig. 2), thereby separating some of the bound fluorophores from the bases. This unexpected excision reaction before optical development desynchronizes the entire flow cell sequencing process, resulting in sequencing errors. Therefore, during reagent recovery, how to avoid cross-contamination caused by the reagent from the previous reaction flowing back into the flow cell is an urgent technical problem that needs to be solved.

In addition, existing recovery solutions all collect the recovered reagent directly into the reagent storage container. Since the reagent inevitably mixes with the liquid that previously occupies the flow cell and its first pipeline during the forward entry into the flow cell, its concentration after being recovered in the reverse direction will inevitably decrease. As a result, the reagent is repeatedly diluted during multiple recovery and reuse, so the effective concentration of the reagent that has reaction in the flow cell cannot be guaranteed. When these recovered reagents enter the storage cells or containers, they further dilute the unused reagents in the containers. For reaction systems in which concentration is positively correlated with reaction efficiency, diluted reagents may significantly reduce the reaction efficiency in the flow cell. Further, the decrease in reagent concentration is positively correlated to the number of recovery times. For example, if the reagent concentration is reduced by 10% each time of recovery, the concentration will be reduced by 34% after four cycles. Therefore, the higher the recovery ratio, the lower the overall reaction efficiency within the flow cell. Therefore, how to guarantee the concentration of reagents involved in biochemical reactions is also a problem that needs to be solved.

In order to avoid cross-contamination caused by the reagent from the previous reaction flowing back into the flow cell, the present application provides a technical solution of arranging a shunt channel at the second end (the lower-reach end when the reagent flows forward) of the flow cell C1 to shunt the reagent that need to be recovered and the reagent from the previous reaction, thereby avoiding cross-contamination.

Referring to Fig. 3, the flow path system according to an embodiment of the present application includes at least two reagent storage containers (not shown), a flow cell C1, a shunting module and a fluid power unit (not shown), wherein the at least two reagent storage containers are configured to store at least two different reagents respectively. The flow cell C1 is configured to accommodate samples, and the flow cell C1 is fluidly connected to the at least two reagent storage containers. The shunting module includes a shunt structure C2 and at least two shunt channels. The shunt structure C2 has a converging port communicating with the flow cell C1 and at least two shunt ports corresponding to the at least two shunt channels. The fluid power unit is connected to the shunting module. The fluid power unit is selectively in fluid communication with one of the at least two shunt channels, and the fluid power unit is configured to drive a forward flow of the reagent from the reagent storage container toward the shunting module. And the fluid power unit is further configured to drive a reverse flow of the reagent from the shunting module toward the reagent storage container.

As shown in Fig. 3, the flow path system according to the embodiment of the present application includes at least two reagent storage containers (not shown), a flow cell C1, a first pipeline L1 connected to a first end of the flow cell C1, a second pipeline L2 connected to a second end of the flow cell C1, a shunt structure C2, and at least two shunt channels. Specifically, FIG. 3 shows that at least two shunt channels include a first shunt channel L3 and a second shunt channel L4.

It should be noted that the first pipeline L1 is configured to be fluidly connected to the reagent storage container, and a component for reagent selection, such as a changeover valve, can also be provided between the first pipeline L1 and the reagent storage container. Similarly, the first shunt channel L3 and the second shunt channel L4 may further be fluidly connected to other functional modules, such as valves for flow path control and fluid power units for driving fluid. The shunt structure C2 may be a simple three-way component (such as a T-shaped three-way component or a Y-shaped three-way component), or a control component such as a three-way solenoid valve, or a combination thereof. In addition, according to the needs of actual applications, the shunt structure C2 can be shunted into more than two branches, and each branch can be shunted into more branches through one or more shunting modules. These downwardly extending bypass designs all fall within the scope of the present application.

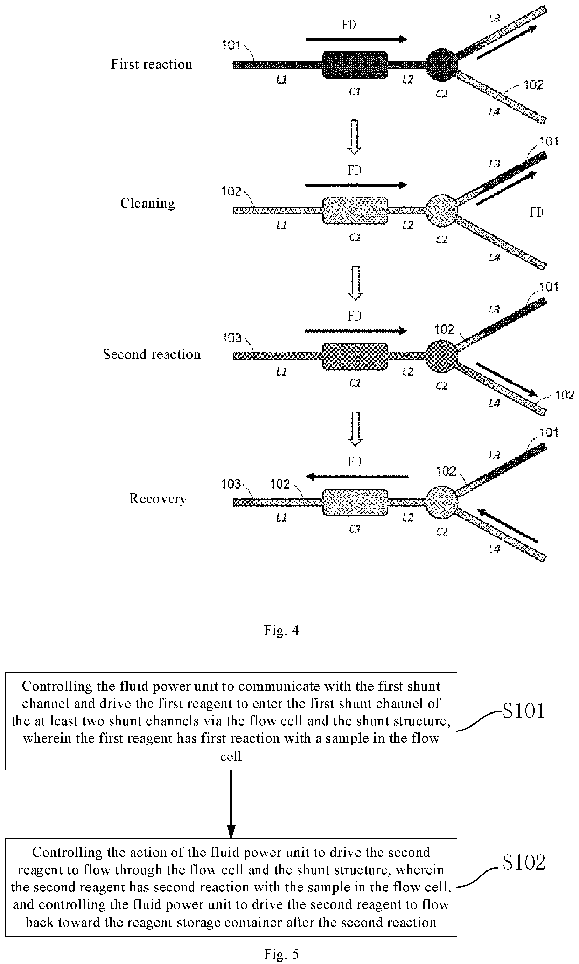

Based on the flow path system design in Fig. 3, a set of reagent recovery logic can be established to avoid cross-contamination in the flow cell C1, as shown in Fig. 4. For ease of understanding, only two reaction steps are involved here, namely the first reaction and the second reaction. It also should be noted that in Fig. 4, only the reagent used in the second reaction is recovered to simplify the discussion, but this does not mean that the reagent used in the first reaction cannot be recovered. Referring to Fig. 4, the entire recovery logic mainly includes the following steps:
S1: The reagent selection component located at the upper reach of the first pipeline L1 is switched so that the first pipeline L1 is fluidly connected to the reagent storage container containing the first reagent 101 (the reagent selection component and the reagent storage container are not marked in Fig. 4) and the fluid power unit is connected to the first shunt channel L3, and then, the first reagent 101 is driven by the fluid power unit to sequentially replace the buffer 102 in the first pipeline L1, the flow cell C1, the second pipeline L2 and the shunt structure C2, and finally flows out along the first shunt channel L3, and in this case, the parabolic interface between the first reagent 101 and the buffer 102 is located in the first shunt channel L3.
S2: At the end of S1, the first reagent 101 in the flow cell C1 immediately has the first reaction with the sample fixed on the flow cell C1.
S3: After the first reaction is completed, the reagent selection component is switched so that the first pipeline L1 is fluidly connected to the buffer storage container containing the buffer 102, and the buffer 102 is driven by the fluid power unit to sequentially clean the first reagent 101 in the first pipeline L1, the flow cell C1, the second pipeline L2, and the shunt structure C2, and finally flows out along the first shunt channel L3, and in this case, the interface between the buffer 102 and the first reagent 101 is located in the first shunt channel L3, ensuring that the first reagent 101 completely leaves the shunt structure C2.
S4: The reagent selection component is switched so that the first pipeline L1 is fluidly connected to the reagent storage container containing the second reagent 103 and the fluid power unit is connected to the second shunt channel L4, and then, the second reagent 103 is driven by the fluid power unit to sequentially replace the buffer 102 in the first pipeline L1, the flow cell C1, the second pipeline L2 and the shunt structure C2, and finally flows out along the second shunt channel L4, and in this case, the parabolic interface between the second reagent 103 and the buffer 102 is located in the second shunt channel L4 and the second reagent 103 in the shunt structure C2 does not mix with the first reagent 101 in the first shunt channel L3.
S5: At the end of S4, the second reagent 103 in the flow cell C1 immediately has the second reaction with the sample fixed on the flow cell C1.
S6: After the second reaction is completed, the fluid power unit reversely drives the second reagent 103 to sequentially leave the second shunt channel L4, the shunt structure C2, the second pipeline L2 and the flow cell C1 to realize the recovery of the second reagent 103.

S1 to S6 describe the recovery strategy of a single reagent (i.e., the second reagent 103) in a two-step reaction system. By delivering the first reagent 101 and the second reagent 103 to the first shunt channel L3 and the second shunt channel L4 respectively and setting a buffer between the first reagent 101 and the second reagent 103, when the second reagent 103 is recovered, the first reagent 101 will not flow back into the flow cell, thereby avoiding the reoccurrence of the first reaction.

It should be noted here that the recovery method shown in Fig. 4 shows the recovery process of the second reagent 103, but in another possible reagent recovery method, the first reagent 101 may also be recovered after the first reaction is completed. For example, after the first reaction, the fluid power unit reversely drives the first reagent 101 to sequentially leave the first shunt channel L3, the shunt structure C2, the second pipeline L2 and the flow cell C1 to realize the recovery of the first reagent 101. For the cyclic reaction, precisely because the second reagent 103 in the previous cycle of reaction flows into the second shunt channel L4, when the first reagent 101 is recovered, the second reagent 103 will not flow back to the flow cell C1, thereby avoiding cross-contamination. In summary, it can be seen that the flow path system of the present application allows the first reagent and the second reagent to enter different shunt channels through the shunting module, as a result, when a certain reagent is recovered, the other reagent will not flow back into the flow cell. That is, the flow path system of the present application can recover both the first reagent and the second reagent, not only the second reagent.

In the above embodiment, in order to allow the first reagent 101 and the second reagent 103 to enter different shunt channels respectively, the fluid power unit needs to be able to make a selection between the first shunt channel and the second shunt channel, thereby ensuring that when the liquid in one channel flows, the liquid in the other channel remains stationary. That is, the fluid power unit is configured to be selectively fluidly connected to one of the at least two shunt channels. Moreover, in the above embodiments, a reagent needs to flow forward into the flow cell to react. When the reagent needs to be recovered, the reagents also need to flow reversely from the shunting module. The bidirectional flow of the reagent as described above is achieved by the fluid power unit. The fluid power unit is configured to drive a drive forward flow of the reagent from the reagent storage container toward the shunting module. And the fluid power unit is further configured to drive a reverse flow of the reagent from the shunting module toward the reagent storage container. Specifically, the fluid power unit may include a power device capable of both forward driving and reverse driving. The fluid power unit may also include a forward power device capable of forward driving and a reverse power device capable of reverse driving.

In summary, it can be seen that in the technical solution of the embodiment of the present application, by arranging a shunting module that includes a shunt structure and at least two shunt channels. In this way, the reagent that needs to be recovered can enter a different shunt channel than the reagent from the previous reaction, thereby avoiding cross-contamination caused by the reagent used in the previous reaction being recovered into the flow cell.

Referring to Fig. 5, an embodiment of the present application further provides a reagent recovery method. The reagent recovery method includes the following steps:
S101: controlling the fluid power unit to fluidly connect with the first shunt channel L3 and drive the first reagent 101 to enter the first shunt channel L3 of the at least two shunt channels via the flow cell C1 and the shunt structure C2, wherein the first reagent 101 has first reaction with a sample in the flow cell C1; and
S102: controlling the action of the fluid power unit to drive the second reagent 103 to flow through the flow cell C1 and the shunt structure C2, wherein the second reagent 103 has second reaction with the sample in the flow cell C1, and controlling the fluid power unit to drive the second reagent 103 to flow back toward the reagent storage container after the second reaction.

In some embodiments, the flow path system further includes a buffer storage container for storing a buffer, the buffer storage container is connected to the flow cell, and the fluid power unit is configured to drive a forward flow of the buffer from the buffer storage container toward the shunting module.

In some embodiments, the reagent recovery method further includes, after the first reaction, controlling the action of the fluid power unit to drive the buffer to flow through the flow cell and the shunt structure and enter the first shunt channel to implement cleaning; after cleaning, controlling the fluid power unit to connect with the second shunt channel and drive the second reagent to enter the second shunt channel of the at least two shunt channels via the flow cell and the shunt structure, and after the second reaction, controlling the fluid power unit to connect with the second shunt channel and drive the second reagent to flow back toward the reagent storage container. That is, in this embodiment, a buffer cleaning process is provided between the first reaction and the second reaction, and the first reagent and the second reagent are allowed to enter two different shunt channels respectively.

In other embodiments, a buffer cleaning process may not be provided between the first reaction and the second reaction. The operation of controlling the action of the fluid power unit to drive the second reagent to flow through the flow cell and the shunt structure, wherein the second reagent has second reaction with the sample in the flow cell, and controlling the fluid power unit to drive the second reagent to flow back toward the reagent storage container after the second reaction includes: controlling the fluid power unit to connect with the first shunt channel and drive the second reagent to enter the first shunt channel via the flow cell and the shunt structure, and after the second reaction, controlling the fluid power unit to connect with the second shunt channel and drive the second reagent to flow back toward the reagent storage container. That is, after the first reaction, the second reagent directly replaces the first reagent in the flow cell and finally flows out along the first shunt channel. In this case, the interface between the first reagent and the second reagent is located in the first shunt channel. The recovery of the second reagent is switched to the second shunt channel.

In some embodiments, the at least two shunt channels and the at least two reagent storage containers are provided in one-to-one correspondence. That is, each reagent enters a different shunt channel, thus preventing other reagents from flowing back into the flow cell during recovery.

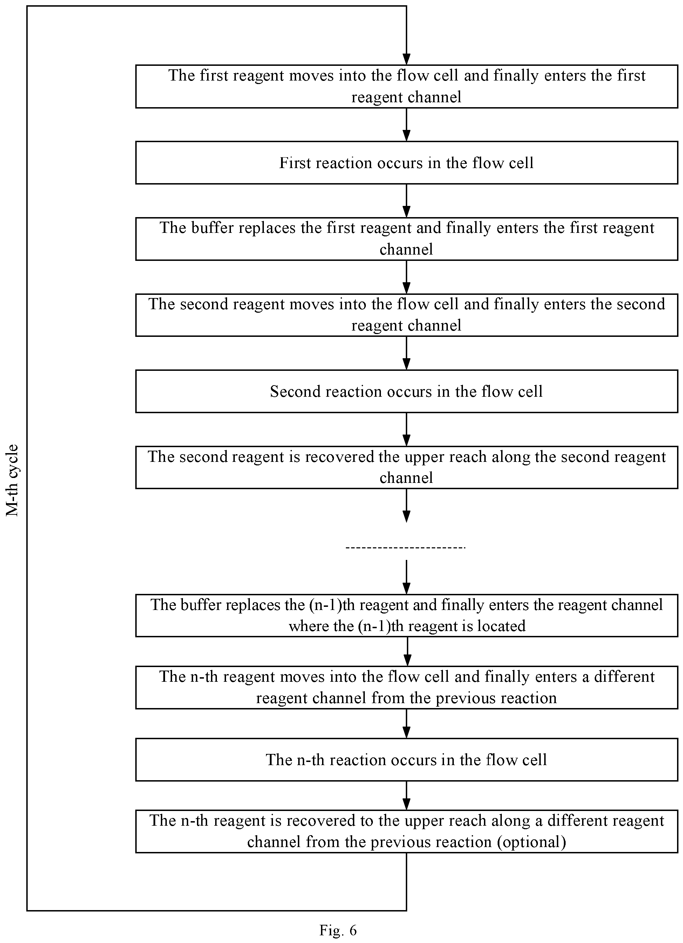

In other embodiments, in order to reduce the size of the flow path system and simplify its structure, the shunting module of the flow path system includes two shunt channels. In a case of cyclic reactions involving two reagents, one reagent is controlled to flow into the first shunt channel, and the other reagent is controlled to flow into the second shunt channel. In other words, the reagents can be recovered according to the recovery strategy shown in Fig. 6. In a case of cyclic multi-reactions involving multiple reagents, the reagent used in each step needs to enter a different shunt channel from the reagent used in the previous reaction to prevent the reagent used in the previous reaction from flowing back into the flow cell and causing out-of-order reactions.

In one possible situation, when one reagent is recovered, not only the reagent from the previous reaction may flow back into the flow cell, but also the reagents from two or more prior reactions may flow back into the flow cell. This can be solved by increasing the volume of the buffer between reagents, reducing the recovery rate, or adding more reagent channels after the shunting module. For example, at least two shunt channels and at least two reagent storage containers mentioned in the above embodiments are provided in one-to-one correspondence, so that each reagent enters a corresponding shunt channel, thereby avoiding cross-contamination.

The inventor of the present application has also conducted in-depth research on the problem of how to ensure the concentration of reagents participating in biochemical reactions while recovering the reagents. In some embodiments, the fluid power unit is configured to drive a reverse flow of the reagent from the shunting module toward the reagent storage container and back into a pipeline connected to an outlet end of the reagent storage container. For example, in the case of recovery of the second reagent, the operation of controlling the fluid power unit to drive the second reagent to flow back toward the reagent storage container includes: controlling the fluid power unit to drive the second reagent to flow back toward the reagent storage container so that the recovered second reagent flows back into a pipeline connected to an outlet end of the reagent storage container. That is, the recovered second reagent only flows back into the pipeline connected to the outlet end of the reagent storage container, and does not return to the reagent storage container. In this way, it can be ensured that the reagent in the reagent storage container is not diluted.

Specifically, the recovery ratio can be adjusted so that the recovered reagent will not return to the reagent storage container. For example, it can be realized by controlling the volume of the recovered reagent.

Of course, if necessary, it is also allowed to return the recovered reagent in part or completely to the reagent storage container, as long as the diluted reagent in the reagent storage container meets the requirement for reuse.

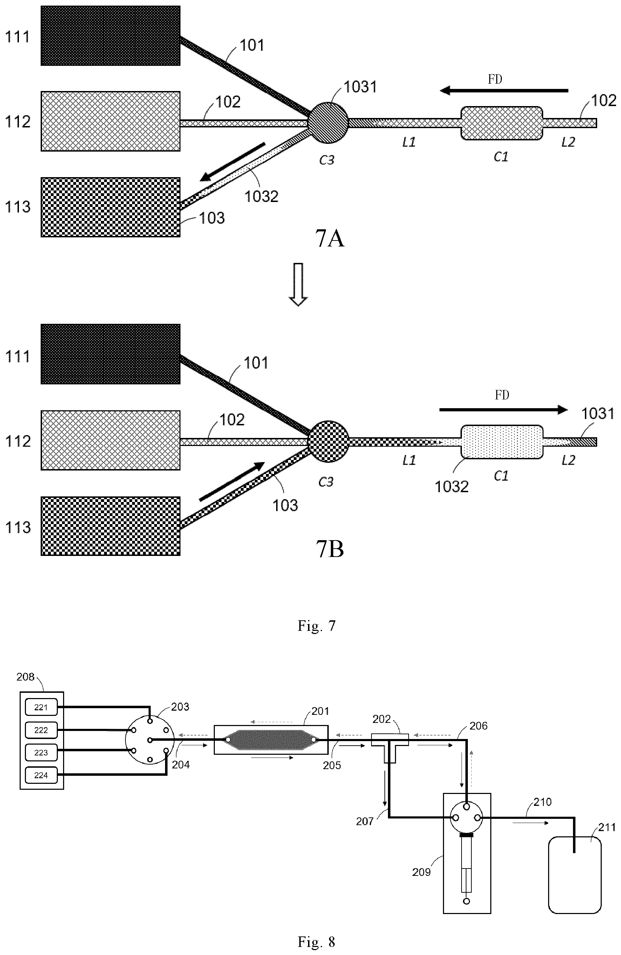

Referring to Fig. 7, the flow path system includes a first storage container 111, a second storage container 112, a third storage container 113, a reagent selection component C3, a first pipeline L1, a flow cell C1, and a second pipeline L2. The first storage container 111 is configured to store a first reagent 101, the second storage container 112 is configured to store a buffer 102, and the third storage container 113 is configured to store a second reagent 103. Fig. 7A shows the recovery process of the current cycle, and Fig. 7B shows the second reaction of next cycle. As shown in Fig. 7A, after the recovery is completed, a certain volume of the second reagent 103 is recovered to the upper reach of the flow cell C3, including the part 1031 that is diluted due to direct contact with the buffer 102 and the part 1032 that is almost undiluted. Since the diluted part 1031 is at the lower reach of the undiluted part 1032, as shown in Fig. 7B, when the second reagent 103 is used in the next cycle, it is certain that the diluted part 1031 first participates in the reagent replacement in the first pipeline L1 and the flow cell C1. With a high flow rate, when the diluted part 1031 passes through the flow cell, it will only initiate a weak second reaction (almost negligible). As the second reagent 103 keeps flowing to the lower reach, the almost undiluted part 1032 enters the flow cell, and in this case, the flow is stopped and this part fully reacts with the sample in the flow cell.

The technical solution of the present application realizes efficient reagent recovery and reuse through flow path system design and recovery logic, which can greatly reduce the amount of reagents consumed in closed pipelines and channels, thereby significantly reducing the cost of reagent consumables in medical testing instruments. In the meanwhile, the present application mainly has the following two advantages:
1) By arranging a shunting module, the reagent that needs to be recovered is shunted from the previous reagent, thus solving the problem of cross-contamination in the flow cell after reagent recovery, preventing complex cyclic orderly reaction systems in precision instruments such as gene sequencers from being disrupted by reagent recovery, and ensuring the quality of biochemical reactions. In a case where the volume of the flow cell is small as compared with the volumes of pipelines at upper and lower reaches, reagent recovery easily causes cross-contamination. This solution makes it possible to achieve high recovery rates in small flow cells.
2) Recovery of reagents will not cause a significant reduction in the overall concentration of the reagent in the storage container. The low-concentration part of the recovered reagent is mainly used to replace another liquid in the flow cell and the common pipeline at its upper reach, rather than participating in a biochemical reaction. The biochemical reaction is still carried out at a high reagent concentration, ensuring the efficiency of the reaction.

Referring Fig. 8, in some embodiments, the at least two shunt channels include a first shunt channel 206 and a second shunt channel 207, the shunt structure includes a three-way pipe 202, and the three-way pipe 202 includes a converging port fluidly connected to the flow cell, a first shunt port connected to the first shunt channel 206, and a second shunt port connected to the second shunt channel 207.

The flow path system of the gene sequencer according to first embodiment shown in Fig. 8 is taken as an example for detailed description. As shown in Fig. 8, the flow path system includes storage containers 208, a reagent selection component 203, a first pipeline 204, a sequencing slide 201, a second pipeline 205, a three-way pipe 202, a first shunt channel 206, a second shunt channel 207, an injection pump 209, a third pipeline 210, and a waste liquid cell 211.

The storage container 208 includes a first storage container for storing a synthesis reagent 221, a second storage container for storing a scanning reagent 222, a third storage container for storing an excision reagent 223, and a fourth storage container for storing a buffer 224. The sequencing slide 201 has a flow cell.

The reagent selection component 203 is configured to selectively fluidly connect the flow cell with one of the storage containers 208, thereby allowing the corresponding reagent to enter the flow cell. The reagent selection component 203 includes a common hole and at least two branch holes, the at least two branch holes are correspondingly connected to the at least two reagent storage containers, the common hole is connected to the flow cell through the first pipeline 204, and the common hole is selectively connected to one of the at least two branch holes. Specifically, the reagent selection component 203 is configured as a reagent selector valve.

The three-way pipe 202 forms a shunt structure. The three-way pipe 202 includes a converging port fluidly connected to the flow cell, a first shunt port connected to the first shunt channel 206, and a second shunt port connected to the second shunt channel 207. Specifically, the three-way pipe 202 is a T-shaped three-way pipe. In other embodiments, it may also be a Y-shaped three-way component.

In other embodiments, the shunt structure may also include a multi-way pipe, such as a four-way pipe, to achieve more shunt channels.

The injection pump 209 forms a fluid power unit. The injection pump 209 has three power ports, the first power port is fluidly connected to the first shunt channel 206, the second power port is fluidly connected to the second shunt channel 207, and the third power port is fluidly connected to the waste liquid cell 211 through the third pipeline 210. The injection pump 209 can selectively provide a driving force to one of the three power ports. The injection pump 209 can provide a forward driving force for the forward flow of the reagent and can also provide a reverse driving force for the reverse flow of the reagent. Of course, in other embodiments, the fluid power unit may also include two independently arranged injection pumps. One of the injection pumps is configured to provide a forward driving force to the reagent, and the other injection pump is configured to provide a reverse driving force to the reagent.

The working process of the flow path system according to the first embodiment shown in Fig. 8 will be described in detail below. In the figure, the solid arrows indicate the flow direction of the reagent during forward flow, and the dotted arrows indicate the flow direction of the reagent during reverse recovery. A DNA fragment to be tested is fixed on the surface of the flow cell of the sequencing slide, and the entire sequencing process is a cyclic "synthesis-test-excision" system as described above. To simplify the description, each reaction involves only one reagent, and between two reactions, a buffer is used as a barrier between reagents. It should be noted that the flow cell in this embodiment, having a volume of about 4 µL, is a typical small-volume flow cell. In comparison, the volume of the first pipeline 204 is 4 µL, the total volume of the reagent selection component 203 and the pipeline between the reagent selection component 203 and the storage container 208 is approximately 30 µL, the volume of the second pipeline 205 is 10 µL, and the volumes of the first shunt channel 206 and the second shunt channel 207 are both 100 µL or above. Before sequencing, the reagent in each storage container 208 is pre-filled into the pipeline between the storage container and the reagent selection component 203. Therefore, during the sequencing process, the reagent replacement mainly occurs in the first pipeline 204 and the flow cell of the sequencing slide 201.

In this embodiment, a complete reaction cycle includes the following steps:
1) the reagent selection component 203 is switched to be fluidly connected to the first storage container, the injection pump 209 is switched to be fluidly connected to the first shunt channel 206, and 40 µL of the synthesis reagent 221 is pumped to flow through the sequencing slide 201 at a high flow rate (e.g., 2000 µL/min, the same below). In this step, the synthesis reagent 221 replaces the buffer 224 originally existing in the first pipeline 204 and the flow cell;
2) synthesis reaction is carried out in the flow cell for a period of time;
3) the injection pump 209 pushes 30 µL of the synthesis reagent 221 to the upper reach in the reverse direction along the first shunt channel 206 (see the dotted arrows in Fig. 8), and the diluted part of the synthesis reagent 221 flows back into the pipeline between the reagent selection component 203 and the storage container 208, but not into the storage container 208;
4) the reagent selection component 203 is switched to be fluidly connected with the fourth storage container, the injection pump 209 remains fluidly connected with the first shunt channel 206, and 50 µL of the buffer 224 is pumped to flow through the sequencing slide 201 to replace the residual synthesis reagent 221 in the first pipeline 204 and the flow cell;
5) the reagent selection component 203 is switched to be fluidly connected with the second storage container, the injection pump 209 remains fluidly connected with the first shunt channel 206, and 40 µL of the scanning reagent 222 is pumped to flow through the sequencing slide 201, and then the sequencing slide 201 is tested by an optical system (not shown);
6) the reagent selection component 203 is switched to be fluidly connected with the fourth storage container, and 50 µL of the buffer 224 is pumped by the injection pump 209 to flow through the sequencing slide 201 to replace the scanning reagent 222 in the first pipeline 204 and the flow cell;
7) the reagent selection component 203 is switched to be fluidly connected to the third storage container, the injection pump 209 is switched to be fluidly connected to the second shunt channel 207, and 40 µL of the excision reagent 223 is pumped to flow through the sequencing slide 201.
8) excision reaction is carried out in the flow cell for a period of time;
9) the reagent selection component 203 is switched to be fluidly connected with the fourth storage container, and the injection pump 209 remains fluidly connected with the second shunt channel 207 and 50 µL of the buffer 224 is pumped to flow through the sequencing slide 201 to replace the excision reagent 223 in the first pipeline 204 and the flow cell; and
10) the injection pump 209 is switched to be fluidly connected to the third pipeline 210 and the waste liquid cell 211 to empty the liquid from the injection pump.

In the above steps, 40 µL of the synthesis reagent 221 is first pumped, and then 30 µL is recovered, so the recovery rate is calculated as 30/40=75%, the net consumption is calculated as 40-30=10 µL, and the replacement ratio r relative to the flow cell volume (4 µL) is calculated as 10/4=2.5. If reagent recovery is not performed, the replacement ratio is calculated as 40/4=10. It can be seen that the replacement ratio is reduced to 1/4 of the original after the introduction of the recovery strategy of the present application.

From the perspective of sequencing quality, a high reagent recovery rate does not have a significant impact on the sequencing results. Table 1 compares the sequencing quality indicators of no recovery and 75% recovery. Both are at the same level in terms of total reads and quality value (Q30).

**Table 1 Comparison of seauencing results under no recovery and 75% recovery strategy**

| Indicator (library: *E*. *coli)* | No recovery | 75% recovery |
|---|---|---|
| Total Reads(M) | 54.5 | 55.3 |
| ≥50M is considered as qualified | | |
| Q30 (%) ≥90 is considered as qualified | 93.13 | 92.7 |
| Consumption of reagent (4µL) | 40 | 10 |

In this embodiment, by introducing the T-shaped three-way pipe 202, the first shunt channel 206 and the second shunt channel 207 at the outlet end of the flow cell, the problem of cross-contamination in the flow cell caused by the backflow of the excision reagent 223 due to reagent recovery is successfully solved. By measuring the concentration of the residual excision reagent in the flow cell after recovery, it is found that the residual concentration is 0.7% when the three-way pipe 202 and the shunt channels are not introduced, and the residual concentration after the introduction is negligible. Since a very small amount of excision reagent can cause out-of-order reactions and sequencing errors, the technical solution of the present application is very important for gene sequencing.

In the embodiment shown in Fig. 8, the synthesis reagent 221 enters the first shunt channel 206, and the excision reagent 223 enters the second shunt channel 207. Therefore, during the recovery of the synthesis reagent 221, the problem of cross-contamination caused by the excision reagent 223 flowing back into the flow cell can be avoided. Of course, when necessary, we can also recover the excision reagent 223, which can also avoid the problem of cross-contamination caused by the synthesis reagent 221 flowing back into the flow cell. In addition, in a case of cyclic multi-reactions involving multiple reagents, in some embodiments, the reagent used in each reaction can be made to enter a different shunt channel from the reagent used in the previous reaction. However, the following situation may also occur when a reagent is recovered, reagents used in two or more previous reactions may be in the same shunt channel as the reagent to be recovered, and backflow may occur. This can be avoided by increasing the volume of the buffer between the reagents. In other embodiments, it can be determined according to the type of reagent whether the reagent used in each step should enter a different shunt channel from the reagent used in the previous reaction. The key is that whether an out-of-order reaction occurs when the reagent used in the current reaction enters the same shunt channel as the reagent used in the previous reaction. For example, in the embodiment shown in Fig. 8, involving the synthesis reagent 221, the scanning reagent 222 and the excision reagent 223, the reaction sequence is synthesis-scanning-excision. Since the scanning reagent 222 flowing back into the flow cell does not cause an out-of-order reaction but the excision reagent 223 flowing back into the flow cell will cause an out-of-order reaction, in the above embodiment, the synthesis reagent 221 and the scanning reagent 222 both flow into the first shunt channel 206, and the excision reagent 223 flows into the second shunt channel 207. This arrangement is mainly for allowing the excision reagent 223 to enter a different shunt channel from the scanning reagent 222.

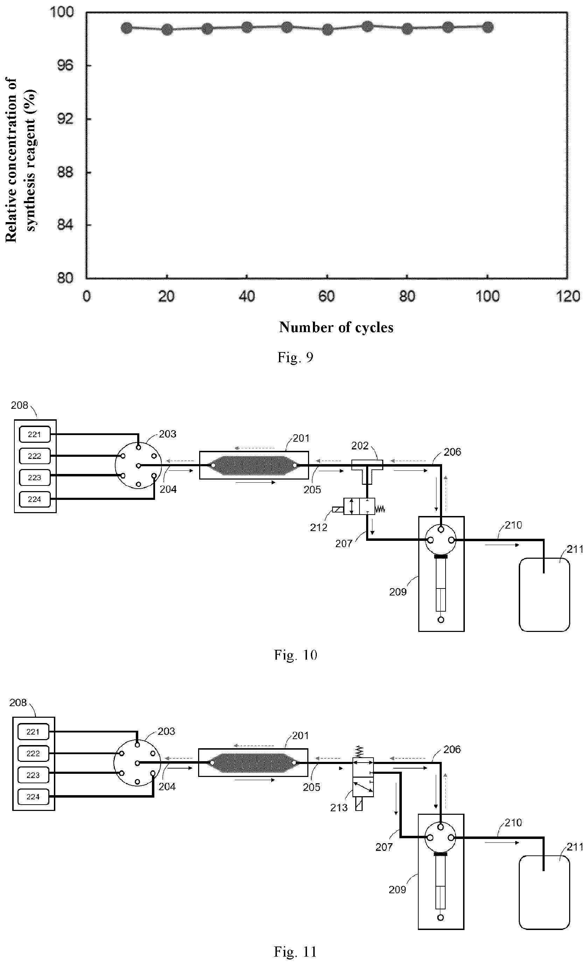

The synthesis reagent in this embodiment is recovered after the reaction and will not be recovered into the storage container 208. In reuse in the next cycle, the diluted part mainly participates in reagent replacement, and the concentration of the reagent participating in the reaction in the flow cell is maintained at a high level. This advantage can be demonstrated by the plot of synthesis reagent concentration versus the number of cycles in the flow cell shown in Fig. 9. It can be seen from the figure that the synthesis reagent concentration remains at an effective relative concentration of 98.7% or above within 100 cycles, which fully illustrates a major advantage of the technical solution described herein.

Referring to Fig. 10, in some embodiments, the shunting module further includes an on-off control valve. The on-off control valve is provided on the first shunt channel 206 and/or the second shunt channel 207. Providing an on-off control valve on the first shunt channel 206 and/or the second shunt channel 207 can strengthen the physical barrier, so that the reagent used in the previous reaction is completely isolated in another shunt channel during reagent recovery.

Specifically, the flow path system of the gene sequencer according to second embodiment shown in Fig. 10 is taken as an example for detailed description.

The change made in the second embodiment, compared to the first embodiment, is that an on-off control valve, specifically a two-position two-way solenoid valve 212, is added to the second shunt channel 207. The solenoid valve functions to strengthen the physical barrier, so that the excision reagent is completely left in the second shunt channel 207 during the recovery of the synthesis reagent. In the design of the embodiment shown in Fig. 8, since the three-way pipe 202, the first shunt channel 206, and the second shunt channel 207 all form passages, when the synthesis reagent is recovered along the first shunt channel 206, the excision reagent in the second shunt channel 207 may still partially flow back into the flow cell due to the unequal flow resistance of the first shunt channel 206 and the second shunt channel 207, bubbles in the pipelines, etc. By introducing the solenoid valve 212, this embodiment can solve this problem. The solenoid valve 212 forms a passage when powered on, and cuts off the passage when powered off.

In this embodiment, the procedure of reagent recovery is a little different from that in the first embodiment, specifically:
1) the reagent selection component 203 is switched to be fluidly connected to the first storage container, the solenoid valve 212 is switched off, the injection pump 209 is switched to be fluidly connected to the first shunt channel 206, and 40 µL of the synthesis reagent 221 is pumped to flow through the sequencing slide 201 at a high flow rate (e.g., 2000 µL/min, the same below). In this step, the synthesis reagent 221 replaces the buffer 224 originally existing in the first pipeline 204 and the flow cell;
2) to 6) are the same as those in the first embodiment;
7) the reagent selection component 203 is switched to be fluidly connected to the third storage container, the solenoid valve 212 is switched on, the injection pump 209 is switched to be fluidly connected to the second shunt channel 207, and 40 µL of the excision reagent 223 is pumped to flow through the sequencing slide 201;
8) to 10) are the same as those in the first embodiment.

In some other embodiments not shown in the drawings, the on-off control valve may also be provided in the first shunt channel 206. Alternatively, an on-off control valves is provided in each of the first shunt channel and the second shunt channel. The on-off control valve may be a solenoid valve or other components that can control the on-off of a pipeline.

Referring to Fig. 11, in some embodiments, the at least two shunt channels include a first shunt channel 206 and a second shunt channel 207. The shunt structure includes a first reversing valve. The first reversing valve has a first port, a second port and a third port, the first port forms a converging port, the second port forms a first shunt port connected to the first shunt channel 206, the third port forms a second shunt port connected to the second shunt channel 207, and the first reversing valve acts to control the connection of the first port to the second port or the third port.

Specifically, the flow path system of the gene sequencer according to third embodiment shown in Fig. 11 is taken as an example for detailed description.

This embodiment is an improved design of the first embodiment. As can be seen from Fig. 11, the only change made in this embodiment, compared to the first embodiment, is to replace the three-way pipe with a reversing valve, specifically a two-position three-way solenoid valve 213. A normally open end of the solenoid valve is fluidly connected to the first shunt channel 206, and a normally closed end is fluidly connected to the second shunt channel 207. Like the second embodiment, this embodiment has a better physical barrier than the first embodiment.

In this embodiment, the procedure of reagent recovery is a little different from that in the first embodiment, specifically:
1) the reagent selection component 203 is switched to be fluidly connected to the first storage container, the solenoid valve 213 remains powered off, the injection pump 209 is switched to be fluidly connected to the first shunt channel 206, and 40 µL of the synthesis reagent 221 is pumped to flow through the sequencing slide 201 at a high flow rate (e.g., 2000 µL/min, the same below). In this step, the synthesis reagent 221 replaces the buffer 224 originally existing in the first pipeline 204 and the flow cell;
2) to 6) are the same as those in the first embodiment;
7) the reagent selection component 203 is switched to be fluidly connected to the third storage container, the solenoid valve 213 is switched off, the injection pump 209 is switched to be fluidly connected to the second shunt channel 207, and 40 µL of the excision reagent 223 is pumped to flow through the sequencing slide 201;
8) to 10) are the same as those in the first embodiment.

In some embodiments, the fluid power unit includes an injection pump 209, the injection pump 209 includes a first power port and a second power port, the first power port is fluidly connected to the first shunt channel, and the second power port is fluidly connected to the second shunt channel.

Specifically, referring to three embodiments shown in Figs. 9, 10 and 11, the injection pump 209 includes a first power port and a second power port, the first power port is fluidly connected to the first shunt channel 206, and the second power port is fluidly connected to the second shunt channel 207.

Referring to three embodiments shown in Figs. 9, 10 and 11, the flow path system further includes a waste liquid cell 211, the injection pump 209 further includes a third power port, and the third power port is connected to the waste liquid cell 211.

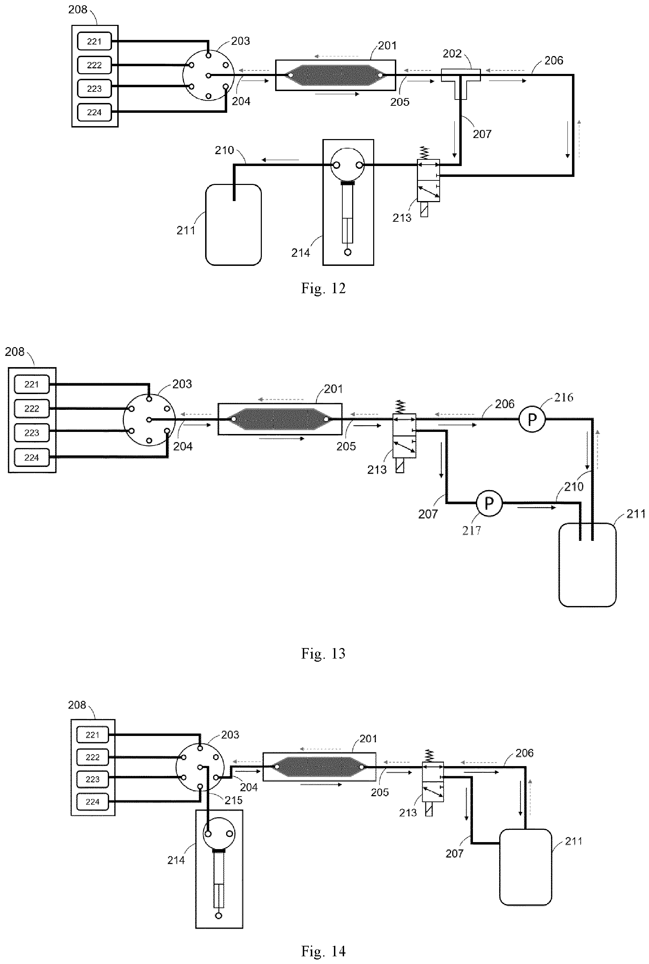

Referring to Fig. 12, in some embodiments, the fluid power unit includes an injection pump 214 and a second reversing valve, the injection pump includes a first power port, the second reversing valve has a first port, a second port and a third port, the first port and the second port are connected to the first shunt channel and the second shunt channel respectively, the third port is connected to the first power port of the injection pump 214, and the second reversing valve acts to control the connection of the third port to the first port or the second port.

The flow path system of the gene sequencer according to fourth embodiment shown in Fig. 12 is taken as an example for detailed description. As can be seen from Fig. 12, the only change made in this embodiment, compared to the first embodiment, is to add a selector value, specifically a two-position three-way solenoid valve 213, to the rear end of the three-way pipe 202. A normally open end of the solenoid valve is fluidly connected to the second shunt channel 207, and a normally closed end is fluidly connected to the first shunt channel 206. In addition, the injection pump 214 in this embodiment has only two optional power ports, one end is fluidly connected to the solenoid valve 213, and the other end is fluidly connected to the third pipeline 210 and the waste liquid cell 211.

In this embodiment, the procedure of reagent recovery is a little different from that in the first embodiment, specifically:
1) the reagent selection component 203 is switched to be fluidly connected to the first storage container, the solenoid valve 213 is switched on, and 40 µL of the synthesis reagent 221 is pumped by the injection pump 214 to flow through the sequencing slide 201 at a high flow rate (e.g., 2000 µL/min, the same below). In this step, the synthesis reagent 221 replaces the buffer 224 originally existing in the first pipeline 204 and the flow cell;
2) synthesis reaction is carried out in the flow cell for a period of time;
3) the injection pump 214 pushes 30 µL of the reagent to the upper reach in the reverse direction along the first shunt channel 206 (see the dotted arrows in Fig. 12 ), and the diluted part of the synthesis reagent 221 flows back into the pipeline between the reagent selection component 203 and the reagent storage container 208, but not into the reagent storage container 208;
4) the reagent selection component 203 is switched to be fluidly connected with the fourth storage container, and 50 µL of the buffer 224 is pumped by the injection pump 214 to flow through the sequencing slide 201 to replace the residual synthesis reagent 221 in the first pipeline 204 and the flow cell;
5) the reagent selection component 203 is switched to be fluidly connected with the second storage container, and 40 µL of the scanning reagent 222 is pumped by the injection pump 214 to flow through the sequencing slide 201, and then the sequencing slide 201 is tested by an optical system (not shown);
6) the reagent selection component 203 is switched to be fluidly connected with the fourth storage container, and 50 µL of the buffer 224 is pumped by the injection pump 214 to flow through the sequencing slide 201 to replace the scanning reagent 222 in the first pipeline 204 and the flow cell;
7) the reagent selection component 203 is switched to be fluidly connected to the excision reagent 223, the solenoid valve 213 is switched off, and 40 µL of the excision reagent 223 is pumped by the injection pump 214 to flow through the sequencing slide 201 and into the second shunt channel 207;
8) excision reaction is carried out in the flow cell for a period of time;
9) the reagent selection component 203 is switched to be fluidly connected with the fourth storage container, and 50 µL of the buffer 224 is pumped by the injection pump 214 to flow through the sequencing slide 201 to replace the excision reagent 223 in the first pipeline 204 and the flow cell; and
10) the injection pump 214 is switched to be fluidly connected to the third pipeline 210 and the waste liquid cell 211 to empty the liquid from the injection pump.

In some embodiments, the flow path system further includes a waste liquid cell 211, the injection pump 214 further includes a second power port, and the second power port of the injection pump 214 is connected to the waste liquid cell. As shown in Fig. 12, the injection pump 214 includes two power ports, one of the power ports is connected to a reversing valve (two-position three-way solenoid valve 213), and the other power port is connected to the waste liquid cell 211 through the third pipeline 210.

In some embodiments, referring to Fig. 13, the fluid power unit includes a first peristaltic pump 216 and a second peristaltic pump 217. The flow path system further includes a waste liquid cell 211, the first shunt channel 206 and the second shunt channel 207 are both connected to the waste liquid cell 211, the first peristaltic pump 216 is provided on the first shunt channel 206, and the second peristaltic pump 217 is provided on the second shunt channel 207.

Fig. 13 shows the flow path system of a gene sequencer according to fifth embodiment. As shown in Fig. 13, this embodiment is a modified design of the third embodiment, and the main change lies in that the injection pump is replaced with two peristaltic pumps 216 and 217. As can be seen from Fig. 13, a normally open end of the solenoid valve 213 is fluidly connected to the first peristaltic pump 216 through the first shunt channel 206, and a normally closed end is fluidly connected to the second peristaltic pump 217 through the second shunt channel 207.

In this embodiment, the procedure of reagent recovery is a little different from that in the third embodiment, specifically:
1) the reagent selection component 203 is switched to be fluidly connected to the first storage container, the solenoid valve 213 remains powered off, and 40 µL of the synthesis reagent 221 is pumped by the injection pump 217 to flow through the sequencing slide 201 at a high flow rate (e.g., 2000 µL/min, the same below). In this step, the synthesis reagent 221 replaces the buffer 224 originally existing in the first pipeline 204 and the flow cell;
2) synthesis reaction is carried out in the flow cell for a period of time;
3) the first peristaltic pump 216 pushes 30 µL of the reagent to the upper reach in the reverse direction along the first shunt channel 206 (see the dotted arrows in Fig. 14), and the diluted part of the synthesis reagent 221 flows back into the pipeline between the reagent selection component 203 and the reagent storage container 208, but not into the reagent storage container 208;
4) the reagent selection component 203 is switched to be fluidly connected with the fourth storage container, and 50 µL of the buffer 224 is pumped by the second peristaltic pump 217 to flow through the sequencing slide 201 to replace the residual synthesis reagent 221 in the first pipeline 204 and the flow cell;
5) the reagent selection component 203 is switched to be fluidly connected with the second storage container, and 40 µL of the scanning reagent 222 is pumped by the second peristaltic pump 217 to flow through the sequencing slide 201, and then the sequencing slide 201 is tested by an optical system (not shown);
6) the reagent selection component 203 is switched to be fluidly connected with the fourth storage container, and 50 µL of the buffer 224 is pumped by the second peristaltic pump 217 to flow through the sequencing slide 201 to replace the scanning reagent 222 in the first pipeline 204 and the flow cell;
7) the reagent selection component 203 is switched to be fluidly connected to the third storage container, the solenoid valve 213 is switched on, and in this case 40 µL of the excision reagent 223 is pumped by the second peristaltic pump 217 to flow through the sequencing slide 201 and into the second shunt channel 207;
8) excision reaction is carried out in the flow cell for a period of time; and
9) the reagent selection component 203 is switched to be fluidly connected with the buffer 224, and 50 µL of the buffer 224 is pumped by the first peristaltic pump 216 to flow through the sequencing slide 201 to replace the excision reagent 223 in the first pipeline 204 and the flow cell.

In some embodiments, the fluid power unit includes an injection pump 214. The flow path system further includes a waste liquid cell 211 and a reagent selection component 203, the first shunt channel 206 and the second shunt channel 207 are both connected to the waste liquid cell 211, the injection pump 214 includes a power port, the reagent selection component 203 includes a common hole and a plurality of branch holes, the common hole is selectively connected to one of the plurality of branch holes, the plurality of branch holes include at least two reagent branch holes correspondingly connected to the at least two reagent storage containers and a flow cell branch hole connected to the flow cell, and the power port of the injection pump 214 is connected to the common hole.

Fig. 14 shows the flow path system of a gene sequencer according to sixth embodiment. This fluid path system is a modified embodiment of the third embodiment shown in Fig. 11. Compared with the third embodiment, the main change made in this embodiment is that the injection pump is placed in front and follows the reagent selection component, so that the forward reagent flow becomes positive-pressure driving. It can be seen from Fig. 14 that the injection pump 214 is fluidly connected to the common hole of the reagent selection component 203 through a pipeline 215, and the common pipeline 204 is instead fluidly connected to a branch hole of the reagent selection component 203. In addition, the shunt channels 206 and 207 of the solenoid valve 213 are both fluidly connected to the waste liquid cell 211. In application, the procedure of reagent recovery is basically the same as that in the third embodiment, except that each time the liquid is pumped forward, the injection pump 214 first pumps the reagent into the pipeline 215 from the common hole of the reagent selection component 203, and the reagent selection component 203 is then switched to be fluidly connected to the first pipeline 204, and finally the injection pump 214 pushes the reagent to the first pipeline 204. In the case of reagent recovery, the injection pump 214 first pumps the used reagent into the pipeline 215 from the first pipeline 204, the reagent selection component 203 is then switched to the hole corresponding to the reagent, and finally the injection pump 214 pushes the recovered reagent to the reagent hole.

The present application further provides a gene sequencer, including a sequencing slide and the flow path system described above, a flow cell being arranged on the sequencing slide.

Finally, it should be explained that the above embodiments are only used to illustrate, rather than to limit, the technical solution of the present application; although the present application has been described in detail with reference to preferred embodiments, those skilled in the art should understand that the specific embodiments of the present application can still be modified or some technical features can be replaced by equivalents, which shall be included in the scope of the technical solution requested by this application without departing from the spirit of the technical solution of this application.

## Claims

1. A flow path system, comprising:
at least two reagent storage containers for storing at least two different reagents respectively;
a flow cell for accommodating samples, and the flow cell being fluidly connected to the at least two reagent storage containers;
a shunting module, comprises a shunt structure and at least two shunt channels, wherein the shunt structure having a converging port communicating with the flow cell and at least two shunt ports corresponding to the at least two shunt channels; and
a fluid power unit fluidly connected to the shunting module, wherein the fluid power unit selectively being in fluid communication with one of the at least two shunt channels, and the fluid power unit being configured to drive a forward flow of the reagent from the reagent storage container toward the shunting module, and the fluid power unit being further configured to drive a reverse flow of the reagent from the shunting module toward the reagent storage container.

2. The flow path system according to claim 1, wherein the at least two shunt channels and the at least two reagent storage containers are provided in one-to-one correspondence.

3. The flow path system according to claim 1, wherein the at least two shunt channels comprise a first shunt channel and a second shunt channel, the shunt structure comprises a three-way pipe, and the three-way pipe comprises a converging port fluidly connected to the flow cell, a first shunt port fluidly communicated with the first shunt channel, and a second shunt port fluidly communicated with the second shunt channel.

4. The flow path system according to claim 3, wherein the shunt structure further comprises an on-off control valve, and the on-off control valve is provided on the first shunt channel and/or the second shunt channel.

5. The flow path system according to claim 1, wherein the at least two shunt channels comprise a first shunt channel and a second shunt channel, the shunt structure comprises a first reversing valve, the first reversing valve has a first port, a second port and a third port, the first port of the first reversing valve forms a converging port, the second port of the first reversing valve forms a first shunt port fluidly communicated with the first shunt channel, the third port of the first reversing valve forms a second shunt port fluidly communicated with the second shunt channel, and the first reversing valve controls the communication of the first port of the first reversing valve to the second port of the first reversing valve or the third port of the first reversing valve.

6. The flow path system according to any one of claims 3-5, wherein the fluid power unit comprises an injection pump, the injection pump comprises a first power port and a second power port, the first power port is fluidly connected to the first shunt channel, and the second power port is fluidly connected to the second shunt channel.

7. The flow path system according to claim 6, wherein the flow path system further comprises a waste liquid cell, the injection pump further comprises a third power port, and the third power port is fluidly communicated with the waste liquid cell.

8. The flow path system according to claim 3, wherein the fluid power unit comprises an injection pump and a second reversing valve, the injection pump comprises a first power port, the second reversing valve has a first port, a second port and a third port, the first port and the second port of the second reversing valve are connected to the first shunt channel and the second shunt channel respectively, the third port of the second reversing valve is connected to the first power port of the injection pump, and the second reversing valve controls the communication of the third port of the second reversing valve to the first port of the second reversing valve or the second port of the second reversing valve.

9. The flow path system according to claim 8, wherein the flow path system further comprises a waste liquid cell, the injection pump further comprises a second power port, and the second power port of the injection pump communicates with the waste liquid cell.

10. The flow path system according to claim 5, wherein the fluid power unit comprises a first peristaltic pump and a second peristaltic pump, the flow path system further comprises a waste liquid cell, the first shunt channel and the second shunt channel both communicates with the waste liquid cell, the first peristaltic pump is provided on the first shunt channel, and the second peristaltic pump is provided on the second shunt channel.

11. The flow path system according to any one of claims 1-10, wherein the flow path system further comprises a reagent selection component, the reagent selection component comprises a common hole and at least two branch holes, the at least two branch holes are correspondingly fluidly connected to the at least two reagent storage containers, the common hole is fluidly connected to the flow cell, and the common hole is selectively communicated with one of the at least two branch holes.

12. The flow path system according to claim 5, wherein the fluid power unit comprises an injection pump, the flow path system further comprises a waste liquid cell and a reagent selection component, the first shunt channel and the second shunt channel both communicates with the waste liquid cell, the injection pump comprises a power port, the reagent selection component comprises a common hole and a plurality of branch holes, the common hole selectively communicates with one of the plurality of branch holes, the plurality of branch holes comprise at least two reagent branch holes correspondingly communicating with the at least two reagent storage containers and a flow cell branch hole communicating with the flow cell, and the power port of the injection pump is connected to the common hole.

13. The flow path system according to any one of claims 1-11, wherein the flow path system further comprises a buffer storage container for storing a buffer, the buffer storage container is fluidly connected to the flow cell, and the fluid power unit is configured to drive a forward flow of the buffer fluid from the buffer storage container toward the shunting module.

14. The flow path system according to claim 1, wherein the fluid power unit is configured to drive a reverse flow of the reagent from the shunting module toward the reagent storage container and back into a pipeline connected to an outlet end of the reagent storage container.

15. A gene sequencer, comprising a sequencing slide and the flow path system according to any one of claims 1-14, the flow cell being arranged on the sequencing slide.

16. A reagent recovery method of the flow path system according to any one of claims 1-14, wherein the at least two different reagents comprise a first reagent and a second reagent, the at least two shunt channels comprising a first shunt channel and a second shunt channel, the reagent recovery method comprising the following steps:
controlling the fluid power unit to communicate with the first shunt channel and drive the first reagent to enter the first shunt channel of the at least two shunt channels via the flow cell and the shunt structure, wherein the first reagent has first reaction with a sample in the flow cell; and
controlling the action of the fluid power unit to drive the second reagent to flow through the flow cell and the shunt structure, wherein the second reagent has second reaction with the sample in the flow cell, and controlling the fluid power unit to drive the second reagent to flow back toward the reagent storage container after the second reaction.

17. The reagent recovery method according to claim 16, wherein the reagent recovery method further comprises, after the first reaction, controlling the action of the fluid power unit to drive the buffer to flow through the flow cell and the shunt structure and enter the first shunt channel to implement cleaning.

18. The reagent recovery method according to claim 17, wherein controlling the action of the fluid power unit to drive the second reagent to flow through the flow cell and the shunt structure, wherein the second reagent has second reaction with the sample in the flow cell, and controlling the fluid power unit to drive the second reagent to flow back toward the reagent storage container after the second reaction comprises: controlling the fluid power unit to communicate with the second shunt channel and drive the second reagent to enter the second shunt channel of the at least two shunt channels via the flow cell and the shunt structure, and after the second reaction, controlling the fluid power unit to communicate with the second shunt channel and drive the second reagent to flow back toward the reagent storage container.

19. The reagent recovery method according to claim 16, wherein controlling the action of the fluid power unit to drive the second reagent to flow through the flow cell and the shunt structure, wherein the second reagent has second reaction with the sample in the flow cell, and controlling the fluid power unit to drive the second reagent to flow back toward the reagent storage container after the second reaction comprises: controlling the fluid power unit to communicate with the first shunt channel and drive the second reagent to enter the first shunt channel via the flow cell and the shunt structure, and after the second reaction, controlling the fluid power unit to communicate with the second shunt channel and drive the second reagent to flow back toward the reagent storage container.

20. The reagent recovery method according to claim 16, further comprising after the first reaction, controlling the fluid power unit to communicate with the first shunt channel and drive the first reagent to flow back toward the reagent storage container, so that the recovered first reagent flows back into a pipeline connected to an outlet end of the reagent storage container that stores the first reagent.

21. The reagent recovery method according to claim 16, wherein controlling the fluid power unit to drive the second reagent to flow back toward the reagent storage container comprises: controlling the fluid power unit to drive the second reagent to flow back toward the reagent storage container, so that the recovered second reagent flows back into a pipeline connected to an outlet end of the reagent storage container.
